# EUROPEAN PATENT SPECIFICATION

(11) **EP 1 405 605 B1**
(45) Date of publication and mention of the grant of the patent: **22.03.2006**
(21) Application number: 03028909.4
(22) Date of filing: 07.06.2001
(51) Int. Cl.: A61B 17/58, A61B 17/88

(54) **Instrument for cranial flap clamp**
Instrument für Schädelkalottenfixiereinrichtung
Instrument pour clamp de volet cranien

(30) Priority: 27.07.2000 US 221148 P
(43) Date of publication of application: 07.04.2004
(62) Divisional of application: 01933533.0
(73) Proprietor: Synthes AG Chur, 7002 Chur (CH)
(72) Inventor: Manthorp, John H., Downington, PA 19335 (US); Hearn, James P., Coatesville, PA 19320 (US); Kerr, Sean H., Collegeville, PA 19426 (US)
(74) Representative: Lusuardi, Werther

(56) References cited:
- DE-C- 19 832 798
- FR-A- 2 777 449
- US-A- 4 050 464

## Description

The present invention is directed to an instrument for a cranial flap clamp for attaching a bone flap to a skull according to the preamble of claim 1.

Craniotomies are surgical procedures performed in the treatment of various brain problems, such as tumors, aneurysms, blood clots, head injuries, abscesses, and the like. During a craniotomy procedure, access to the brain is achieved by the creation of a hole in the bone that defines the skull. The hole or "window" in the skull is usually created by identifying the area of the brain to which access is needed, drilling several holes into the skull near the periphery of this area, inserting a cutting tool into one of the holes, and making cuts from one hole to another. Removing the cut-out area of the skull, generally referred to as a bone flap, allows the desired access to the brain.

If all of the drilled holes are joined by cuts, such that the cuts form a complete outline of the "window", then the bone flap can simply be removed. Alternatively, if the cuts form only a partial outline of the window, the bone flap can be bent out of the way, in a hinge-like manner. Although the size and shape of the bone flap will vary with the desired cranial access area and size, a typical bone flap would be generally rectangular in shape and approximately four by six centimeters.

After the desired medical or surgical procedure on the brain has been performed, the bone flap must be replaced and held in a stable position to allow the skull to heal. There are many methods available for affixing the bone flap to the skull. One general method, for example, requires drilling pairs of holes in the edges of the skull and bone flap, threading wire through the holes, and twisting or tying the ends of the wire together to secure the edges of the bone flap to the skull. Disadvantages of this method include the tedious nature and length of time required for the procedure and the possibility of injury from drilling the holes too deep or from the sharp ends of the wires.

Another method of fixation generally involves the use of bone plates which are secured across the gaps between the bone flap and skull by screws. The disadvantages associated with the use of plates and screws relate to the undesirable cosmetic appearance resulting from the protrusion of the plate and screw above the bone surface. As there is minimal intervening soft tissue between the skull and the skin, unappealing external appearance is particularly a problem. The lack of soft tissue also has the unwanted consequence of permitting the patient to feel the plate and screw simply by pressing on the scalp.

From DE-C 198 32 798 LERCH an instrument for applying a cranial flap clamp is known according to the preamble of claim 1. This known tool has the disadvantage that the extension member may not be crimped after the two clamping members of the cranial flap clamp have been positioned.

Therefore no tool is available for the surgeon with which he can easily crimp an extension member of a cranial flap clamp consisting of two clamping members and an extension member.

On this point, the invention intends to provide remedial measures. The invention is based on the objective of providing a securing instrument that firstly allows to move the clamping members of the cranial flap clamp from a first (non-clamping) to a second (clamping) position and secondly allows to crimp the extension member which connects the two clamping members in-situ by means of a grimping assembly being mounted on the same securing instrument.

The invention solves the posed problems with an instrument for a cranial flap clamp that displays the features of claim 1.

The present invention relates to a securing instrument for use with a cranial flap clamp. The securing instrument has first and second pivotally connected handles, a gripping arm operatively connected with the first handle, and a tensioning arm operatively connected with the second handle. The gripping and tensioning arms are movable in response to movement of the first and second handles and a slot extends through the distal portions of the gripping and tensioning arms for receiving the extension member of the cranial flap clamp. The securing instrument also includes a clamping element operatively associated with the slot. The clamping element has an inactive configuration, allowing sliding of the extension member through the slot, and an active configuration, clamping a portion of the extension member against a wall of the slot to inhibit sliding of the extension member through the slot. A crimping assembly is operatively associated with the arms for crimping the extension member.

In use, pivoting of the first and second handles causes the gripping and tensioning arms to separate with the tensioning arm engaging the outer surface of the external (second) clamping member and the clamping element in the active position, thereby moving the internal (first) and second clamping members from the first position to the second position.

The clamping element can be a clamp rotatably coupled to the gripping arm so that rotation of the clamp within the slot upon separation of the gripping and tensioning arms moves the clamping element from the inactive configuration to the active configuration. Furthermore, a resilient member, such as a spring, can bias the clamping element in the active configuration when the gripping and tensioning arms are separated. The tensioning arm can include a foot with a ramped surface maintaining the clamping element in the inactive configuration when the gripping and tensioning arms are in contact.

In an exemplary embodiment, the crimping assembly comprises a slider having a crimping edge for crimping the extension member and sides configured and dimensioned for sliding in a grooved end of the tensioning arm, and a link operatively associated with the tensioning arm for sliding movement with respect thereto. The link has a distal end coupled to the slider and a proximal end with teeth. A lever is rotatably coupled to the tensioning arm and has a distal end with teeth engaging the teeth of the distal end of the link. The crimping assembly can also include a cutting stop that cooperates with the crimping edge of the slider to crimp and cut the extension member.

In order to isolate the distal end of the securing instrument so that only the distal end is in contact with the cranium, both the gripping and tensioning arms can have a curved or angled intermediate portion so that the distal portion of the gripping arm extends from the intermediate portion substantially parallel to the proximal portion.

A resilient element can be placed between the first and second handles, thereby biasing the first and second handles away from each other. In order to maintain the first and second handles at a given position, the securing instrument can include a locking bar having a first end pivotably coupled to the first handle and a curved body portion with a plurality of teeth and a locking clip pivotably coupled to the second handle and having a through channel. The locking clip is movable between a free position in which the locking bar is moveable in and out of the channel and a ratchet position in which the teeth of the locking bar engage an edge of the channel to prohibit the locking bar from moving out of the channel.
FIG. 1 is side view of a securing instrument for use with a cranial flap clamp;
FIG. 2 is a cross sectional view of the distal portion of the securing instrument of FIG. 1;
FIG. 3 is a cross sectional view of the proximal portion of the gripping arm of the securing instrument of FIG. 1; and
FIG. 4 a side view of a cranial flap clamp for use with the securing instrument of FIGs. 1 to 3.

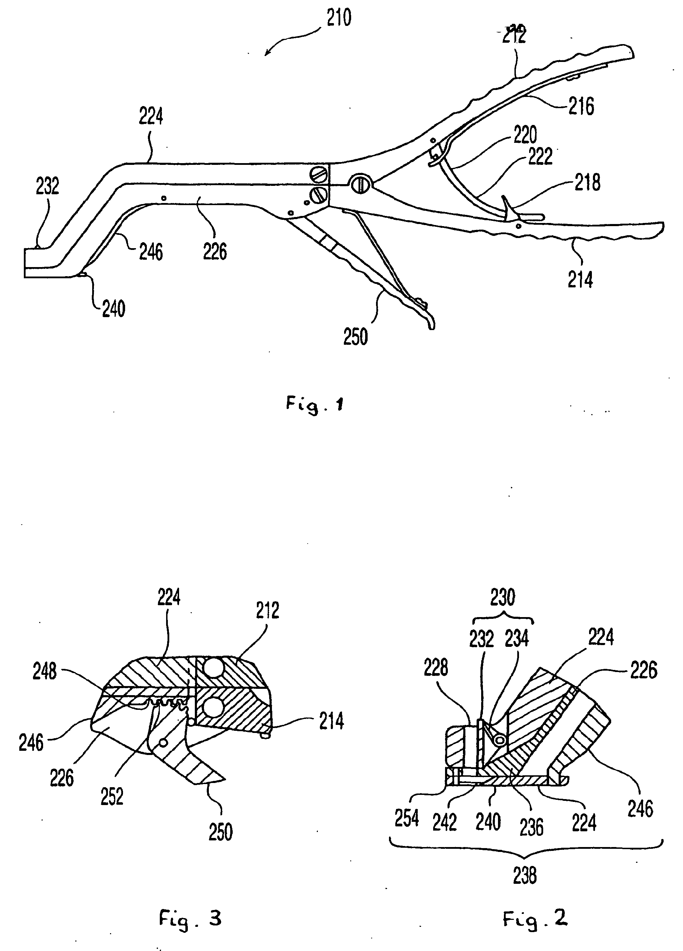

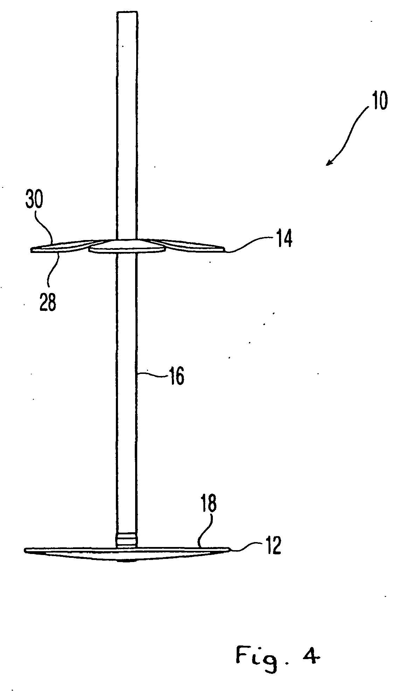

The present invention relates to an instrument for use with a cranial flap clamp 10 (Fig. 4) for fixing a bone flap to a skull. Typically, such clamps include a first clamping member 12, an extension member 16, and a second clamping member 14. Preferably, at least a portion of the inner surface 18 of the first clamping member 12 is positionable against inferior surfaces of the bone flap and skull and at least a portion of the inner surface 28 of the second clamping member 14 is positionable against superior surfaces of the bone flap and skull. The extension member 16 extends from the first clamping member 12 and is configured and dimensioned to fit between the bone flap and the skull. The second clamping member 14 has an opening through its inner and outer surfaces 28;30 for slidably receiving the extension member 16.

Securing instrument 210 includes first and second handles 212, 214. First and second handles 212, 214 are pivotably connected such that upon squeezing, the distal ends of first and second handles 212, 214 spread apart from each other. A resilient element 216, such as a leaf spring, is located between first and second handles 212, 214 and biases their proximal ends away from each other so that upon releasing of the squeezing pressure, the distal ends of first and second handles 212, 214 pivot back toward each other until contact.

A locking mechanism can be provided to resist the biasing force of resilient element 216. For example, a locking clip 218 is located on second handle 214 and is movable between a free position in which a locking bar 220 is free to move through a channel in locking clip 218 and a ratchet position in which locking bar 220 can only move through locking clip 218 in one direction. This ratchet mechanism allows first and second handles 212, 214 to maintain their relative positions after squeezing and release of the squeezing pressure. In order to create the ratchet effect, a portion of locking bar 220 can be provided with teeth 222 that engage an edge of the channel when locking clip 218 is in the ratchet position.

A gripping arm 224 is operatively connected with first handle 212 and a tensioning arm 226 is operatively connected with second handle 214. Gripping and tensioning arms 224, 226 are movable in response to movement of the first and second handles. Thus, as first and second handles 212, 214 are squeezed, gripping and tensioning arms 224, 226 separate or spread apart from each other.

A slot 228 extends through the distal portions of gripping and tensioning arms 224, 226 for receiving the extension member 16 of the cranial flap clamp 10. Gripping and tensioning arms 224, 226 can be made as straight extensions from the distal ends of their respective handle. In an exemplary embodiment, however, each of gripping and tensioning arms 224, 226 has a curved body portion with the distal end of securing instrument 210 isolated from the rest of the instrument, so that in use, only the distal end of securing instrument 210 is in contact with the cranium.

A clamping element 230 is operatively associated with slot 228. Clamping element 230 has an inactive configuration in which extension member 16 can freely slide through slot 228 and an active configuration in which a portion of extension member 16 is clamped against a wall of slot 228 to inhibit sliding of the extension member 16 through slot 228. Clamping element 230 includes a clamp 232 rotatably coupled to gripping arm 224. Rotation of clamp 232 within slot 228 upon separation of gripping and tensioning arms 224, 226 moves clamping element 230 from the inactive configuration to the active configuration. A resilient member 234 biases clamping element 230 in the active configuration when gripping and tensioning arms 224, 226 are separated. Tensioning arm 226 includes a foot 236 with a ramped surface maintaining clamping element 230 in the inactive configuration when gripping and tensioning arms 224, 226 are in contact.

In order to crimp the extension member 16 after proper positioning, a crimping assembly 238 is operatively associated with tensioning arm 226. Alternatively, crimping assembly 238 can be associated with gripping arm 224. In an exemplary embodiment, a slider 240 has a crimping edge 242 for crimping the extension member 16 and sides 244 configured and dimensioned for sliding in a grooved end of tensioning arm 226. A link 246 is operatively associated with tensioning arm 226 so that link 246 can slide with respect to tensioning arm 226. Link 246 has a distal end coupled to slider 240 and a proximal end with teeth 248. A lever 250 has a distal end rotatably coupled to tensioning arm 226. The distal end of lever 250 is provided with teeth 252 that engage teeth 248 of the distal end of link 246. As lever 250 is pivoted, the engagement of teeth 248, 252 causes the pivoting to be translated to sliding motion of link 246 and slider 240. A leaf spring or other similar mechanism can be used to cause lever 250 to pivot back. Crimping assembly 238 can also include a cutting stop 254 cooperating with crimping edge 242 of slider 240 to crimp and cut the extension member 16.

In use, extension member 16 is inserted into slot 228 and securing instrument 210 is moved down toward the cranium with the cranial flap clamp 10 in the position shown in FIG. 4. First and second handles 212, 214 are pivoted to cause gripping and tensioning arms 224, 226 to move away from each other. This movement causes tensioning arm 226 to push against the outer surface 30 of the second clamping member 14 and clamping element 230 to be in the active position, thereby holding the extension member 16 and drawing the first clamping member 12 toward the second clamping member 14. With the first and second clamping members 12;14 in the second position, crimping assembly 238 can be used to crimp and cut the extension member 16.

The invention described and claimed herein is not to be limited in scope by the specific embodiments herein disclosed, since these embodiments are intended solely as illustrations of several aspects of the invention. The scope of the invention is defined by the claims.

## Claims

1. A securing instrument (210) for a cranial flap clamp (10) comprising:
a) first and second pivotally connected handles (212;214), each of said handles (212;214) having proximal and distal portions;
b) a gripping arm (224) operatively connected with the first handle (212) and having proximal and distal portions;
c) a tensioning arm (226) operatively connected with the second handle (214) and having proximal and distal portions, the gripping and tensioning arms (224;226) movable in response to movement of the first and second handles (212;214);
d) a slot (228) extending through the distal portions of the gripping and tensioning arms (224;226) for receiving the extension member (16) of a cranial flap clamp (10);
**characterized by**
e) a clamping element (230) operatively associated with the slot (228) and having active and inactive configurations, the inactive configuration allowing sliding of an extension member (16) of a cranial flap clamp (10) through the slot (228) and the active configuration clamping a portion of the extension member (16) against a wall of the slot (228) to inhibit sliding of the extension member (16) through the slot (228); and
f) a crimping assembly (238) operatively associated with at least one of the gripping and tensioning arms (224;226) for crimping an extension member (16),
g) wherein pivoting of the first and second handles (212;214) causes the gripping and tensioning arms (224;226) to separate with the tensioning arm (226) engaging the outer surface (30) of an external clamping member (14) of a cranial flap clamp (10) and the clamping element (230) in the active position thereby moving the internal and external clamping members (12;14) of a cranial flap clamp (10) from a non-clamping to a clamping position.

2. The securing instrument of claim 1 wherein the clamping element (230) comprises a clamp (232) rotatably coupled to the gripping arm (224) and wherein rotation of the clamp (232) within the slot (228) upon separation of the gripping and tensioning arms (224;226) moves the clamping element (230) from the inactive configuration to the active configuration.

3. The securing instrument of claim 2 further comprising a resilient member biasing the clamping element (230) in the active configuration when the gripping and tensioning arms (224;226) are separated.

4. The securing instrument of claim 3 wherein the tensioning arm (226) includes a foot maintaining the clamping element (230) in the inactive configuration when the gripping and tensioning arms (224;226) are in contact.

5. The securing instrument of claim 4 wherein the foot includes a ramped surface.

6. The securing instrument of claim 1 wherein the distal portion of the tensioning arm (226) includes a grooved end and the crimping assembly comprises (238):
A) a slider (240) having a crimping edge for crimping the extension member (16) of a cranial flap clamp (10) and sides (244) configured and dimensioned for sliding in the grooved end of the tensioning arm (226);
B) a link (246) operatively associated with the tensioning arm (226) for sliding movement with respect thereto, the link (246) having a distal end coupled to the slider (240) and a proximal end with teeth (248); and
C) a lever (250) having a distal end rotatably coupled to the proximal portion of the tensioning arm (226), the distal end having teeth (252) engaging the teeth (248) of the distal end of the link (246).

7. The securing instrument of claim 6 wherein the crimping assembly (238) includes a cutting stop (254), the cutting stop (254) cooperating with the crimping edge (242) of the slider (240) to crimp and cut the extension member (16) of a cranial flap clamp (10).

8. The securing instrument of claim 1 wherein:
a) the gripping arm (224) further comprises an intermediate portion located between the gripping arm proximal and distal portions with the gripping arm proximal portion extending from the distal portion of the first handle (212), the distal portion of the gripping arm (224) extending from the intermediate portion substantially parallel to the proximal portion, and the intermediate portion angling from the gripping arm proximal portion and;
b) the tensioning arm (226) further comprises an intermediate portion located between the tensioning arm proximal and distal portions with the tensioning arm proximal portion extending from the distal portion of the second handle (214), the distal portion of the tensioning arm (226) extending from the intermediate portion substantially parallel to the proximal portion, and the intermediate portion angling from the tensioning arm proximal portion.

9. The securing instrument of claim 1 further comprising a resilient element (216) located between the first and second handles (212;214) biasing the first and second handles (212;214) away from each other.

10. The securing instrument of claim 9 further comprising:
A) a locking bar (220) having a first end pivotably coupled to the first handle (212) and a curved body portion with a plurality of teeth (222); and
B) a locking clip (218) pivotably coupled to the second handle (214) and having a through channel,
C) wherein the locking clip (218) is movable between a free position in which the locking bar (220) is moveable in and out of the channel and a ratchet position in which the teeth (222) of the locking bar (220) engage an edge of the channel to prohibit the locking bar (220) from moving out of the channel.

## Patentansprüche

1. Befestigungsinstrument (210) für eine Schädeldeckelklemme (10), umfassend:
a) einen ersten und einen zweiten schwenkbar miteinander verbundenen Griff (212;214), wobei ein jeder dieser Griffe (212;214) einen proximalen und einen distalen Abschnitt aufweist;
b) einen Greifarm (224), welcher funktionell mit dem ersten Griff (212) verbunden ist und einen proximalen und einen distalen Abschnitt aufweist;
c) einen Spannarm (226), welcher funktionell mit dem zweiten Griff (214) verbunden ist und einen proximalen und einen distalen Abschnitt aufweist, wobei der Greifarm und der Spannarm (224;226) in Abhängigkeit der Bewegung des ersten und des zweiten Griffs (212;214) bewegbar sind;
d) einen Schlitz (228), welcher sich durch die distalen Abschnitte des Greifarms und des Spannarms (224;226) erstreckt, um das Anziehelement (16) einer Schädeldeckelklemme (10) aufzunehmen;
**gekennzeichnet durch**
e) ein Klemmteil (230), welches funktionell zu dem Schlitz (228) angeordnet ist und über eine aktive und eine inaktive Stellung verfügt, wobei die inaktive Stellung eine Verschiebung des Anziehelements (16) einer Schädeldeckelklemme (10) **durch** den Schlitz (228) hindurch ermöglicht, und die aktive Stellung einen Abschnitt des Anziehelements (16) gegen eine Wand des Schlitzes (228) klemmt, um eine Verschiebung des Anziehelements (16) durch den Schlitz (228) hindurch zu verhindern; und
f) eine Sickeneinrichtung (238), welche mit zumindest einem der Elemente bestehend aus dem Greifarm und dem Spannarm (224;226) in Wirkverbindung steht, um ein Anziehelement (16) zu sicken,
g) wobei ein Verschwenken des ersten und des zweiten Griffs (212;214) bewirkt, dass sich der Greifarm und der Spannarm (224;226) auseinander bewegen, wobei der Spannarm (226) mit der Aussenfläche (30) eines äusseren Klemmelements (14) einer Schädeldeckelklemme (10) in Eingriff tritt und das Klemmteil (230) sich in der aktiven Stellung befindet, wodurch das innere und das äussere Klemmelement (12;14) einer Schädeldeckelklemme (10) von einer nicht klemmenden Stellung in eine klemmende Stellung bewegt werden.

2. Befestigungsinstrument nach Anspruch 1, wobei das Klemmteil (230) eine Klemme (232) umfasst, welche drehbar mit dem Greifarm (224) verbunden ist, und wobei ein Drehen der Klemme (232) innerhalb des Schlitzes (228) nach erfolgter Auseinanderbewegung des Greifarms und des Spannarms (224;226) das Klemmteil (230) von der inaktiven Stellung in die aktive Stellung bewegt.

3. Befestigungsinstrument nach Anspruch 2, welches weiterhin ein elastisches Element umfasst, welches das Klemmteil (230) in der aktiven Stellung vorspannt, wenn der Greifarm und der Spannarm (224;226) auseinandergespreizt werden.

4. Befestigungsinstrument nach Anspruch 3, wobei der Spannarm (226) einen Fuss beinhaltet, welcher das Klemmteil (230) in der inaktiven Stellung hält, wenn der Greifarm und der Spannarm (224, 226) miteinander in Kontakt stehen.

5. Befestigungsinstrument nach Anspruch 4, wobei der Fuss eine schräge Fläche aufweist.

6. Befestigungsinstrument nach Anspruch 1, wobei der distale Abschnitt des Spannarms (226) ein gerilltes Ende beinhaltet und die Sickeneinrichtung (238)folgendes umfasst:
A) einen Schieber (240) mit einer Sickenkante zum Sicken des Anziehelements (16) einer Schädeldeckelklemme (10) und mit Seitenflächen (244), die entsprechend ausgelegt und dimensioniert sind, um in dem gerillten Ende des Spannarms (226) verschiebbar zu sein;
B) ein Verbindungsglied (246), welches in Wirkverbindung mit dem Spannarm (226) steht, um in Bezug auf diesen eine Verschiebebewegung ausführen zu können, wobei das Verbindungsglied (246) ein mit dem Schieber (240) verbundenes, distales Ende, sowie ein mit Zähnen (248) versehenes, proximales Ende aufweist; und
C) einen Hebel (250) mit einem distalen Ende, welches drehbar mit dem proximalen Abschnitt des Spannarms (226) verbunden ist, wobei das distale Ende Zähne (252) aufweist, die mit den Zähnen (248) des distalen Endes des Verbindungsglieds (246) in Eingriff treten.

7. Befestigungsinstrument nach Anspruch 6, wobei die Sickeneinrichtung (238) einen Schneidanschlag (254) beinhaltet, wobei der Schneidanschlag (254) mit der Sickenkante (242) des Schiebers (240) zusammenwirkt, um das Anziehelement (16) einer Schädeldeckelklemme (10) zu sicken und zu beschneiden.

8. Befestigungsinstrument nach Anspruch 1, wobei:
a) der Greifarm (224) weiterhin einen zwischen dem proximalen und dem distalen Abschnitt des Greifarms angeordneten Mittelabschnitt umfasst, wobei der proximale Abschnitt des Greifarms sich von dem distalen Abschnitt des ersten Griffs (212) aus erstreckt, der distale Abschnitt des Greifarms (224) sich von dem Mittelabschnitt aus im wesentlichen parallel zu dem proximalen Abschnitt erstreckt, und der Mittelabschnitt von dem proximalen Abschnitt des Greifarms aus einen Winkel bildet; und
b) der Spannarm (226) weiterhin einen zwischen dem proximalen und dem distalen Abschnitt des Spannarms angeordneten Mittelabschnitt umfasst, wobei der proximale Abschnitt des Spannarms sich von dem distalen Abschnitt des zweiten Griffs (214) aus erstreckt, der distale Abschnitt des Spannarms (226) sich von dem Mittelabschnitt aus im wesentlichen parallel zu dem proximalen Abschnitt erstreckt, und der Mittelabschnitt von dem proximalen Abschnitt des Spannarms aus einen Winkel bildet.

9. Befestigungsinstrument nach Anspruch 1, welches weiterhin ein elastisches Element (216) umfasst, das zwischen dem ersten und dem zweiten Griff (212, 214) angeordnet ist, wodurch der erste und der zweite Griff (212;214) unter Vorspannung auseinander gespreizt werden.

10. Befestigungsinstrument nach Anspruch 9, welches weiterhin folgendes umfasst:
A) einen Verriegelungsstab (220) mit einem schwenkbar mit dem ersten Griff (212) verbundenen ersten Ende und einem mit einer Mehrzahl von Zähnen (222) versehenen, gekrümmten Hauptabschnitt; und
B) eine Verriegelungsklammer (218), welche schwenkbar mit dem zweiten Griff (214) verbunden ist und einen durchgehenden Kanal aufweist,
C) wobei die Verriegelungsklammer (218) zwischen einer freien Stellung, in welcher der Verriegelungsstab (220) in den Kanal hinein und aus diesem heraus bewegt werden kann, und einer Sperrstellung, in welcher die Zähne (222) des Verriegelungsstabs (220) mit einer Kante des Kanals in Eingriff treten, um zu verhindern, dass der Verriegelungsstab (220) sich aus dem Kanal herausbewegt, verstellbar ist.

## Revendications

1. Instrument de fixation (210) pour un dispositif de serrage pour couvercle crânien (10), lequel comprend :
a) une première et une deuxième poignée (21;214) reliées l'une à l'autre de manière pivotante, chacune de ces poignées (212;214) présentant une partie proximale et une partie distale;
b) un bras de préhension (224) coopérant avec la première poignée (212) et présentant une partie proximale et une partie distale;
c) un bras tendeur (226) coopérant avec la deuxième poignée (214) et présentant une partie proximale et une partie distale, le bras de préhension et le bras tendeur (224;226) pouvant être déplacés en fonction du mouvement de la première et de la deuxième poignée (212;214);
d) une fente (228) s'étendant à travers les parties distales du bras de préhension et du bras tendeur (224;226) afin de recevoir l'élément tendeur (16) du dispositif de serrage pour couvercle crânien (10);
**caractérisé par**
e) un organe de serrage (230) qui coopère avec la fente (228) et qui dispose d'une position active et d'une position inactive, la position inactive permettant un coulissement de l'élément tendeur (16) d'un dispositif de serrage pour couvercle crânien (10) à travers la fente (228) et la position active bloquant une partie de l'élément tendeur (16) contre une paroi de la fente (228) afin d'empêcher un coulissement de l'élément tendeur (16) à travers la fente (228); et
f) un dispositif à border (238) qui coopère avec au moins un des éléments constitués par le bras de préhension et le bras tendeur (224;226) afin de border un élément tendeur (16),
g) un pivotement de la première et de la deuxième poignée (212;214) faisant s'éloigner l'un de l'autre le bras de préhension et le bras tendeur (224;226), le bras tendeur (226) entrant en prise avec la surface extérieure (30) d'un élément de serrage extérieur (14) d'un dispositif de serrage pour couvercle crânien (10) et l'organe de serrage (230) se trouvant dans la position active, ce qui fait passer les éléments de serrage intérieur et extérieur (12;14) d'un dispositif de serrage pour couvercle crânien (10) d'une position non bloquante à une position bloquante.

2. Instrument de fixation selon la revendication 1, l'organe de serrage (230) comprenant une pince (232) reliée au bras de préhension (224) de manière à pouvoir tourner, et en ce que le fait de faire tourner la pince (232) à l'intérieur de la fente (228), une fois que le bras de préhension et le bras tendeur (224;226) ont été éloignés l'un de l'autre, fait passer l'organe de serrage (230) de la position inactive à la position active.

3. Instrument de fixation selon la revendication 2 qui comprend en outre un élément élastique qui met en précontrainte l'organe de serrage (230) en position active lorsque le bras de préhension et le bras tendeur (224;226) sont écartés l'un de l'autre.

4. Instrument de fixation selon la revendication 3, le bras tendeur (226) comportant un pied qui maintient l'organe de serrage (230) dans la position inactive lorsque le bras de préhension et le bras tendeur (224;226) sont en contact.

5. Instrument de fixation selon la revendication 4, le pied présentant une surface inclinée.

6. Instrument de fixation selon la revendication 1, la partie distale du bras tendeur (226) comportant une extrémité rainurée et le dispositif à border (238) comprenant :
A) un coulisseau (240) pourvu d'une arête à border permettant de border l'élément tendeur (16) d'un dispositif de serrage pour couvercle crânien (10) et de faces latérales (244) qui sont conçues et dimensionnées de manière à pouvoir coulisser dans l'extrémité rainurée du bras tendeur (226);
B) un organe de liaison (246) qui coopère avec le bras tendeur (226) afin de pourvoir réaliser un mouvement coulissant par rapport à celui-ci, l'organe de liaison (246) présentant une extrémité distale reliée au coulisseau (240) ainsi qu'une extrémité proximale pourvue de dents (248); et
C) un levier (250) comportant une extrémité distale qui est reliée à la partie proximale du bras tendeur (226) de manière à pouvoir tourner, l'extrémité distale présentant des dents (252), lesquelles entrent en prise avec les dents (248) de l'extrémité distale de l'organe de liaison (246).

7. Instrument de fixation selon la revendication 6, le dispositif à border (238) comportant une butée de coupe (254), la butée de coupe (254) coopérant avec l'arête à border (242) du coulisseau (240) afin de border et de couper l'élément tendeur (16) d'un dispositif de serrage pour couvercle crânien (10).

8. Instrument de fixation selon la revendication 1,
a) le bras de préhension (224) comprenant en outre une partie centrale disposée entre la partie proximale et la partie distale dudit bras de préhension, la partie proximale du bras de préhension s'étendant depuis la partie distale de la première poignée (212), la partie distale du bras de préhension (224) s'étendant, depuis la partie centrale, essentiellement parallèlement à la partie proximale et la partie centrale formant, depuis la partie proximale dudit bras de préhension, un angle donné; et
b) le bras tendeur (226) comprenant en outre une partie centrale disposée entre la partie proximale et la partie distale dudit bras tendeur, la partie proximale du bras tendeur s'étendant depuis la partie distale de la deuxième poignée (214), la partie distale du bras tendeur (226) s'étendant, depuis la partie centrale, essentiellement parallèlement à la partie proximale et la partie centrale formant, depuis la partie proximale dudit bras tendeur, un angle donné.

9. Instrument de fixation selon la revendication 1, lequel comprend en outre un élément élastique (216) disposé entre la première et la deuxième poignée (212;214), grâce auquel la première et la deuxième poignée (212;214) sont écartées l'une de l'autre avec une précontrainte donnée.

10. Instrument de fixation selon la revendication 9, lequel comprend en outre :
A) une tige de verrouillage (220) présentant une première extrémité reliée de manière pivotante à la première poignée (212) et une partie principale courbe pourvue d'une pluralité de dents (222); et
B) une pince de verrouillage (218) reliée de manière pivotante à la deuxième poignée (214) et présentant un canal traversant,
C) la pince de verrouillage (218) pouvant être déplacée entre une position libre permettant de faire entrer la tige de verrouillage (220) dans le canal et de l'en faire ressortir, et une position de blocage, dans laquelle les dents (222) de la tige de verrouillage (220) entrent en prise avec une arête du canal afin d'empêcher la tige de verrouillage (220) de sortir du canal.
